# EUROPÄISCHE PATENTANMELDUNG

(11) **EP 3 243 547 A1**
(43) Veröffentlichungstag der Anmeldung: **15.11.2017**
(21) Anmeldenummer: 17169510.9
(22) Anmeldetag: 04.05.2017
(51) Int. Cl.: A61M 25/01, A61M 25/10, A61B 90/00

(54) **BALLONKATHETER MIT RÖNTGENSICHTBAREM MARKER**

(30) Priorität: 12.05.2016 DE 102016108783
(71) Anmelder: Biotronik AG, 8180 Bülach (CH)
(72) Erfinder: Fargahi, Amir, 8180 Bülach (CH)
(74) Vertreter: Randoll, Sören

(57) **Zusammenfassung**

Die Anmeldung betrifft ein Ballonkatheter (10) mit einem expandierbaren Ballon (12), einem in dem Ballon (12) angeordneten Innenschaft (13), und mindestens einem Marker (15, 16, 31, 32), wobei der mindestens eine Marker (15, 16, 31, 32) ein röntgensichtbares Material aufweist. Um eine hohe Röntgensichtbarkeit unter Vermeidung von scharfen Kanten an dem Röntgenmarker zu erzielen und eine exakte Positionierung des Röntgenmarkers zu erreichen, ist das röntgensichtbare Material als Beschichtung auf und/oder in einem Polymerträger (14, 30) ausgebildet und der Polymerträger (14, 30) auf dem Innenschaft (13) befestigt. Es wird ferner ein einfaches und kostengünstiges Verfahren zum Herstellen eines derartigen Ballonkatheters (10) beschrieben.

## Beschreibung

Die Erfindung betrifft einen Ballonkatheter mit einem expandierbaren Ballon, einem in dem Ballon angeordneten Innenschaft und mindestens einem Marker, wobei der mindestens eine Marker ein röntgensichtbares Material aufweist. Die Erfindung betrifft außerdem ein Verfahren zur Herstellung eines solchen Ballonkatheters.

Ein Ballonkatheter ist ein Werkzeug zur minimalinvasiven Erweiterung oder Wiedereröffnung von verengten oder verschlossenen Blutgefäßen, wie sie beispielsweise durch Kalkablagerungen im Gefäß hervorgerufen werden. Bei der Angioplastie (PTA), insbesondere der perkutanen transluminalen koronaren Angioplastie (PTCA), wird ein Ballonkatheter meistens von der Leiste aus über einen Führungsdraht in der verengten Stelle platziert und mit einem Druck von mehreren Bar beaufschlagt. Durch den Druck wird der Ballon in dem Gefäß dilatiert und drückt von Innen gegen die Gefäßwand beziehungsweise die Kalkablagerungen. Dadurch ist es oftmals möglich, die verengte Stelle wieder aufzuweiten. Um ein genaues Einführen und Positionieren des Katheters im Körper zu ermöglichen, wird die Position des Ballons mittels eines Röntgengerätes beobachtet. Dazu besitzt der Ballonkatheter Marker (Markierungen) aus einem röntgensichtbaren Material. Beispielsweise wird an beiden Enden des Ballons je ein Marker angebracht, was den zusätzlichen Vorteil hat, dass neben der Position auch die Ausrichtung des Ballonkatheters beobachtet werden kann.

Typische Ballonkatheter weisen einen expandierbaren Ballon auf, der einen Innenschaft umgibt. Als röntgensichtbare Marker werden meist runde Platin- oder Goldhülsen verwendet. Um diese auf dem Innenschaft des Ballonkatheters zu befestigen, wird in der Regel ein Rundhämmerprozess (Swaging) eingesetzt. Die Befestigung des Markers mittels Rundhämmern erweist sich aus mehreren Gründen als nicht zufriedenstellend.

Durch das Rundhämmern können am Marker scharfe Kanten entstehen, welche zur Perforation des Ballons und dadurch zu einem nicht funktionsfähigen Ballon führen können. Bereits bei kleinsten Beschädigungen in der Wandung kann die Druckbeaufschlagung beeinträchtigt sein, sodass der Ballon entweder nicht vollständig dilatiert werden oder nicht mit dem nötigen Druck auf die Gefäßwand einwirken kann. Bei der durch das Rundhämmern bedingten Krafteinwirkung auf den Marker kann es außerdem vorkommen, dass der Marker während des Befestigungsprozesses verschoben wird. Dadurch ist sowohl die genaue Position eines Markers als auch der Abstand zwischen dem proximalen und dem distalen Marker nicht exakt einzuhalten. Dies kann zu derart großen Abweichungen führen, dass ein Ballonkatheter nicht zu gebrauchen ist. Die Krafteinwirkung kann zudem dazu führen, dass ein innerer, zur Aufnahme des Führungsdrahts dienender Kanal des Innenschafts verengt wird. Darüber hinaus ist eine Automatisierung des Rundhämmerns schwierig. Die Herstellung mittels Rundhämmern ist somit zeitaufwändig, kostenintensiv und der hiermit hergestellte Ballonkatheter entspricht häufig nicht den vorgegebenen Qualitätsanforderungen. Es ist außerdem nachteilig, dass derartige Metallhülsen eine große Steifigkeit besitzen, die das Bewegen des Katheters beim Einbringen in das zu behandelnde Gefäß erschwert.

Alternativ können Polymermarker eingesetzt werden, die beispielsweise aus einem Polymermaterial, wie bspw. Nylon, Urethan oder thermoplastischen Elastomeren, bestehen, welches mit Wolfram gefüllt ist. Nachteilig bei diesen Polymermarkern ist ihre große Dicke, die für die ausreichende Röntgensichtbarkeit erforderlich ist und die den Querschnitt eines Ballonkatheters vergrößert. Auch eine Automatisierung der Herstellung solcher Polymermarker ist nicht einfach möglich.

Aufgabe der vorliegenden Erfindung ist es daher, einen Ballonkatheter zu schaffen, der eine hohe Röntgensichtbarkeit aufweist, wobei scharfe Kanten an den Röntgenmarkern vermieden werden und wobei die Röntgenmarker an einer exakten Position angeordnet sind. Die Aufgabe besteht ferner darin, ein einfaches und kostengünstiges Verfahren zur Herstellung eines solchen Ballonkatheters anzugeben.

Die obige Aufgabe wird gelöst durch einen Ballonkatheter mit den Merkmalen des Anspruchs 1.

Erfindungsgemäß ist das röntgensichtbare Material als Beschichtung auf und/oder in einem Polymerträger ausgebildet, der auf dem Innenschaft befestigt ist. Die Beschichtung kann vollständig oder zumindest teilweise aus dem röntgensichtbaren Material bestehen.

Die Beschichtung mit dem röntgensichtbaren Material kann beispielsweise lediglich auf der Oberfläche des Polymerträgers angeordnet sein. Im Rahmen der Erfindung ist es jedoch auch möglich, die Beschichtung im Bereich einer Vertiefung oder Aussparung des Polymerträgers aufzubringen. Hierdurch kann die Dicke des Markers vergrößert werden, was zu einer verbesserten Röntgensichtbarkeit führt. Weiterhin ist es möglich, dass das röntgensichtbare Material beim Aufbringen zumindest teilweise in den Polymerträger hinein diffundiert.

Eine erfindungsgemäße Beschichtung auf einem Polymerträger weist herstellungsbedingt keinerlei scharfe Kanten auf, welche zu einer Beschädigung des Ballons führen könnten. Damit ist sichergestellt, dass der Ballon beim Beaufschlagen mit Druck, beispielsweise im Körper eines Patienten, zuverlässig dilatiert werden kann. Es wird außerdem verhindert, dass durch scharfe Kanten Verletzungen im Inneren des Körpers eines Patienten auftreten können. Im Vergleich mit anderen Methoden kann ein erfindungsgemäßer Marker wesentlich exakter auf dem Polymerträger und damit am Ballonkatheter positioniert werden. Dies erhöht die Genauigkeit, mit der auf einer Röntgenaufnahme die Position des Ballonkatheters bestimmt werden kann. Es wird außerdem vermieden, dass die Befestigung des Markers am Innenschaft zu einer Verengung eines Kanals im Inneren des Innenschafts führt. Da der Innenschaft in der Regel ebenfalls aus einem Polymermaterial gefertigt ist, besteht ein weiterer Vorteil der erfindungsgemäßen Lösung darin, dass der Polymerträger sich einfach auf dem Innenschaft befestigen lässt. Je nach Auswahl des Materials des Polymerträgers weist dieser zusammen mit dem mindestens einen Marker zudem eine größere Flexibilität als eine Metallhülse auf.

In einer bevorzugten Ausführungsform der Erfindung sind ein erster Marker in einem ersten Abschnitt des Polymerträgers und ein zweiter Marker in einem zweiten Abschnitt des Polymerträgers angeordnet. Der erste Marker und der zweite Marker weisen dabei einen genau definierten Abstand voneinander auf. Hierdurch kann in einem Röntgenbild neben der Position des Ballonkatheters auch dessen Ausrichtung bestimmt werden. Durch den genau definierten Abstand kann bei der Beobachtung des Ballonkatheters im Röntgengerät auch genau erkannt werden, über welche Länge eine Aufweitung des Gefäßes durch den Ballonkatheter stattfindet. Der Abstand der Marker wird beispielsweise durch den Abstand der Mittellinien der Marker bestimmt.

In einer weiteren bevorzugten Ausführungsform der Erfindung ist der Polymerträger mit seinem proximalen Ende mittels einer vorzugsweise punktweisen Schweißverbindung am Innenschaft befestigt und/oder der Polymerträger mit seinem distalen Ende mittels einer vorzugsweise umlaufenden, fluiddichten Schweißverbindung ebenfalls am Innenschaft befestigt. Besonders bevorzugt wird der Polymerträger mit seinem proximalen Ende zusammen mit dem Innenschaft und dem Außenschaft gemeinsam verschweißt. Besonders bevorzugt ist der Polymerträger mit seinem distalen Ende mittels einer gleichzeitigen Schweißung von Ballon, Innenschaft und einer Spitze des Ballonkatheters befestigt.

Alternativ zum Verschweißen kann der Polymerträger außen auf den Innenschaft mittels einer Klebeverbindung, d. h. mit einem Klebstoff, befestigt sein.

Es ist weiterhin von Vorteil, wenn der Polymerträger platten- oder bandförmig ausgebildet ist und der Polymerträger zur Befestigung am Innenschaft zumindest teilweise um den Innenschaft gebogen ist. Der Polymerträger kann eine Breite aufweisen, die dem Umfang des Innenschafts entspricht, sodass der Polymerträger einmal rund um den Innenschaft gebogen ist. Ein platten- oder bandförmiger Polymerträger lässt sich einfach herstellen und verarbeiten.

Wenn der Polymerträger bandförmig ausgebildet, seitlich am Innenschaft befestigt ist und lediglich eine Breite aufweist, die deutlich kleiner als der Umfang des Innenschafts ist, so besitzt der Innenschaft auch nach der Befestigung des beschichteten Polymerträgers eine hohe Flexibilität. Dadurch lässt sich der erfindungsgemäße Ballonkatheter einfacher durch enge Gefäßgänge führen.

In einer alternativen Ausführungsform der Erfindung ist der Polymerträger hülsenförmig ausgebildet und konzentrisch um den Innenschaft angeordnet, wobei der Innenschaft an der Innenfläche des Polymerträgers befestigt ist. Dadurch wird der Innenschaft vom Polymerträger in einem Abschnitt ummantelt. Hierdurch kann das Zusammensetzen des Ballonkatheters und Befestigen des beschichteten Polymerträgers weiter vereinfacht werden, da der Polymerträger einfach über den Innenschaft des Ballonkatheters geschoben werden kann. Das Aufbringen des Polymerträgers auf den Innenschaft kann vorzugsweise dadurch vereinfacht werden, dass der hülsenförmige Polymerträger in Richtung seiner Längsachse über seine gesamte Länge oder einen Teil seiner gesamten Länge geschlitzt ist. Der Polymerträger kann nach dem Aufschieben beispielsweise gemeinsam mit dem Ballonkatheter am Innenschaft verschweißt werden.

In einer bevorzugten Ausführungsform der Erfindung ist der Polymerträger aus einem Material gefertigt, das Polyimid und/oder LCP enthält. Ein Ballonkatheter mit einem Polymerträger aus einem dieser beständigen Materialien lässt sich besonders einfach fertigen, da sie besonders gut mit dem Innenschaft verschweißt werden können. Der Polymerträger weist bevorzugt eine Dicke zwischen 10 µm und 50 µm, besonders bevorzugt zwischen 20 µm und 30 µm, auf. Da der Polymerträger lediglich als Trägermaterial für den Marker dient, wird das Polymermaterial möglichst dünn gewählt. Der Polymerträger muss allerdings eine ausreichende Dicke aufweisen, um nicht bei der Herstellung oder bei der Verwendung beschädigt zu werden.

Es ist weiter von Vorteil, als röntgensichtbares Material mindestens ein Material der Gruppe aufweisend Gold, Tantal, Niob, Platin und Legierungen aus diesen Materialien einzusetzen. Insbesondere Gold weist eine besonders hohe Sichtbarkeit unter Röntgenstrahlung auf, lässt sich ausgezeichnet verarbeiten, und ist weitestgehend biokompatibel. Die Beschichtung kann eine Dicke zwischen 20 µm und 100 µm, insbesondere zwischen 40 µm und 60 µm, aufweisen. Ein durch Beschichtung aufgebrachter Marker kann, insbesondere wenn er in einer in dem Polymerträger eingebrachten Ausnehmung angeordnet ist, eine größere Dicke aufweisen als ein mit alternativen Methoden angebrachter Marker, wodurch eine verbesserte Sichtbarkeit des Ballonkatheters im Röntgenbild gegeben ist.

Die obige Aufgabe wird ferner durch ein Verfahren zur Herstellung eines derartigen Ballonkatheters mit den Merkmalen des Anspruchs 8 gelöst.

Erfindungsgemäß beinhaltet das erfindungsgemäße Verfahren die folgenden Schritte:
- Bereitstellen einer Polymerträger-Platte, eines Polymerträger-Bands oder einer Polymerträger-Hülse,
- Auf- und/oder Einbringen einer Beschichtung aus einem röntgensichtbaren Material in Form mindestens eines Markers auf und/oder in der Polymerträger-Platte, dem Polymerträger-Band oder der Polymerträger-Hülse und
- Befestigen der beschichteten Polymerträger-Platte, des beschichteten Polymerträger-Band oder der beschichteten Polymerträger-Hülse auf dem Innenschaft des Ballonkatheters.
Mit dem erfindungsgemäßen Verfahren lassen sich Ballonkatheter mit den obigen Vorteilen einfach und kostengünstig herstellen.

In einer bevorzugten Ausführungsform der Erfindung wird zum Befestigen eines vorzugsweise von einer Polymerträger-Platte, z. B. einer Leiterplatte aufweisend Polyimid und/oder LCP, abgetrennten Polymerträger-Bands auf dem Innenschaft das Polymerträger-Band zunächst auf dem Innenschaft fixiert, dann werden der Innenschaft innerhalb des Ballons angeordnet und anschließend Ballon, Innenschaft und Polymerband an ihrem distalen Ende gleichzeitig miteinander verschweißt. Vorzugsweise werden Ballon, Innenschaft und Polymerträger-Band, jeweils an ihrem distalen Ende gleichzeitig z. B. mittels Laser derart miteinander verschweißt, dass die Verbindung zwischen Ballon und Innenschaft fluiddicht ist. Vorzugsweise kann auch eine Katheterspitze bei dieser Schweißung gleichzeitig mitverschweißt werden. Zur temporären Befestigung der Komponenten im distalen Bereich (Ballon, Innenschaft, gegebenenfalls Spitze, Polymerträger-Band) wird ein Schrumpfschlauch über die Komponenten gezogen, der nach dem Schweißen wieder entfernt wird.

Am proximalen Ende des Polymerträger-Bands wird dieses mittels punktueller Verschweißung gleichzeitig mit der Verschweißung von Innenschaft und Außenschaft z. B. mittels Laser an dem Ballonkatheter befestigt. Der Ballon wird an seinem proximalen Ende mit dem Außenschaft mittels Verschweißen, z. B. mittels Laser, fluiddicht verbunden.

Die Beschichtung auf und/oder in dem Polymerträger kann bevorzugt mittels einer Abscheidung aus der Gasphase, beispielsweise mittels PVD (Physical Vapour Deposition) oder CVD (Chemical Vapour Deposition), erfolgen. Weiterhin sind auch Verfahren zur Abscheidung aus der flüssigen Phase weit verbreitet. Solche Beschichtungsverfahren sind beispielsweise aus der Fertigungstechnik der Elektronikindustrie bekannt. Es existieren daher handelsübliche Apparaturen für diese Techniken, die daher kostengünstig zur Verfügung stehen. Mit diesen Methoden können besonders genaue Beschichtungen mit einer exakt einstellbaren Zusammensetzung auf den Leiterplatten realisiert werden.

Da bei dem erfindungsgemäßen Verfahren zur Herstellung eines Ballonkatheters auf die Beschichtungsmethoden der Elektrotechnik zurückgegriffen wird, ist das gesamte Verfahren automatisierbar. Dies führt zu wesentlich geringeren Herstellungskosten eines Ballonkatheters als mit alternativen Verfahren.

In einer bevorzugten Ausführungsform des erfindungsgemäßen Herstellungsverfahrens weist die Polymerträger-Platte eine Längsrichtung und eine dazu senkrechte Querrichtung auf. Die Beschichtung der Leiterplatte mit dem röntgensichtbaren Material erfolgt dabei in zwei entlang der Querrichtung verlaufenden Streifen, die von der Oberkante der Polymerträger-Platte bis zur Unterkante der Polymerträger-Platte reichen können. Die beiden quer verlaufenden, beschichteten Streifen werden in einem genau definierten Abstand aufgebracht, der dem späteren Abstand der Marker entspricht. Wird nun mit einem Schnitt entlang der Längsrichtung von der beschichteten Polymerträger-Platte ein Band abgeschnitten, so enthält dieses ein Teilstück des ersten Streifens als erster Marker und an einer Position mit definiertem Abstand ein Teilstück des zweiten Streifens als zweiter Marker. Werden weitere Bänder von der Polymerträger-Platte mit der gleichen Breite abgeschnitten, so sind sämtliche Bänder im Wesentlichen identisch zueinander, insbesondere hinsichtlich der Positionierung der Marker. Mit diesem Verfahren können daher automatisiert viele Polymerträger-Bänder mit zwei als Beschichtung aufgebrachten Markern hergestellt werden, welche reproduzierbar den gleichen, exakt festgelegten Abstand zwischen den Markern aufweisen.

Die Erfindung wird nachfolgend anhand von Ausführungsbeispielen und unter Bezugnahme auf die Figuren erläutert. Dabei bilden alle beschriebenen und/oder lediglich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: einen erfindungsgemäßen Ballonkatheter in einem Querschnitt;
- Fig. 2a - 2c: die Schritte der Herstellung eines Polymerträger-Bands jeweils in einer Ansicht von oben und in einer Ansicht von der Seite;
- Fig. 3: einen Polymerträger eines erfindungsgemäßen Ballonkatheters, der als Polymerträger-Hülse ausgeführt ist, in einer perspektivischen Ansicht von der Seite und
- Fig. 4a - 4c: die Schritte der Herstellung eines erfindungsgemäßen Ballonkatheters, jeweils in einer Ansicht von der Seite beziehungsweise in einer Schnittdarstellung.

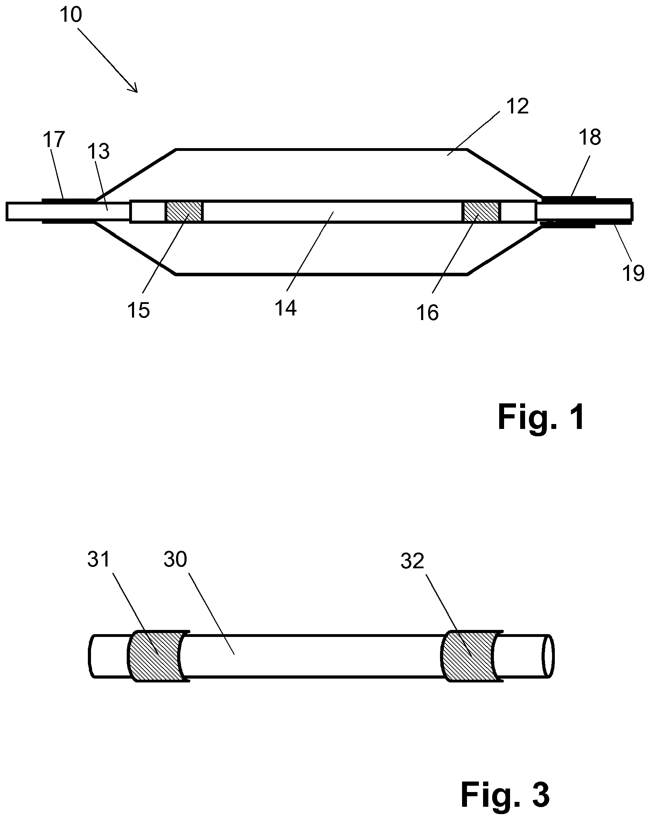

Der erfindungsgemäße Ballonkatheter 10, wie er in Fig. 1 zu sehen ist, weist einen Innenschaft 13 und einem Ballon 12 auf, welcher in einem dilatierten Zustand dargestellt ist. Hierfür wurde der Ballon mit einem Druck von mehreren Bar beaufschlagt, wobei die Druckbeaufschlagung mittels einer Flüssigkeit oder eines Gases erfolgen kann. Als Material für den Ballon kommen alle dafür gängigen Materialien wie Polyethylene (PET), Polycarbonate (PC), Polyimide (PA), PEBAX, PA11, PA12 oder PVC sowie deren Blends in Frage.

Der Ballon 12 umschließt einen Teilabschnitt des Innenschafts 13. In dem umschlossenen Teilabschnitt des Innenschafts ist auf dessen äußeren Umfang ein Polymerträger in Form eines Polymerträger-Bands (im Folgenden auch kurz Polymerband) 14 befestigt. Auf dem Polymerband 14 ist ein distaler Marker 15 und ein proximaler Marker 16 angeordnet. Beide Marker 15, 16 sind in Form einer Beschichtung auf dem Polymerband 14 aufgebracht. Der Ballon 12 ist in einem distalen Bereich 17 mit einem Außenschaft 19 und in einem proximalen Bereich 18 mit dem Innenschaft 13 jeweils fluiddicht verbunden. Der Außenschaft 19 hat die Funktion, den Ballon 12 zu inflatieren bzw. deflatieren. Der Ballon wird daher an seinem proximalen Ende mit dem distalen Ende des Außenschafts 19 fluiddicht verschweißt.

Das Polymerband 14 wird im distalen Bereich 17 mit dem Ballon 12 und dem Innenschaft 13 (und gegebenenfalls mit einer nicht dargestellten Spitze) gleichzeitig verschweißt. Im proximalen Ballon-Bereich 18 erfolgt die Befestigung des Polymerbands 14 gleichzeitig mit dem punktuellen Verschweißen von Innenschaft 13 und Außenschaft 19. Die Befestigung an den beiden Enden des Polymerbands 14 ist ausreichend, da das Polymerband 14 beim Einsatz des Ballonkatheters mechanisch nur gering belastet wird.

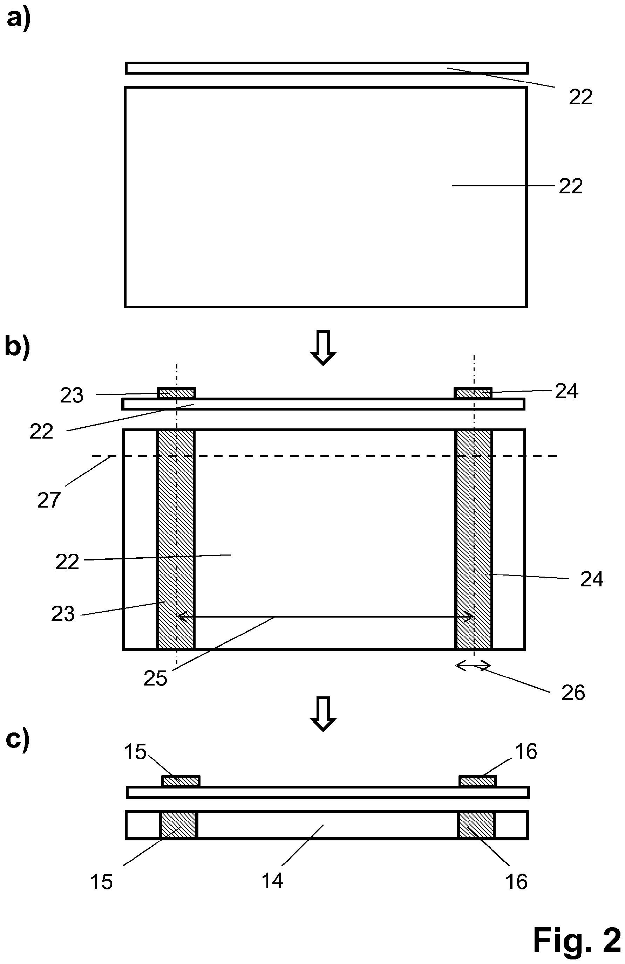

Zur Herstellung eines erfindungsgemäßen Ballonkatheters 10 wird in einem ersten Schritt der mit den Markern 15, 16 beschichtete Polymerträger 14, wie im schematischen Ablauf der Fig. 2a) bis 2c) gezeigt, hergestellt. Dazu wird als Ausgangsmaterial eine in Fig. 2a gezeigte Polymerträger-Platte, beispielsweise eine Leiterplatte 22 aus LCP, verwendet. Die Dicke der Leiterplatte 22 beträgt beispielsweise 25 µm und entspricht der Dicke des späteren Polymerbands 14. Die weiteren Maße der Leiterplatte 22 können in einer Längsrichtung der späteren Länge des Polymerbandes 14 entsprechen und in einer Querrichtung beliebig gewählt werden.

Auf diese Leiterplatte 22 werden mittels bekannter Beschichtungsverfahren aus der Gas- oder Flüssigphase, beispielsweise mittels PVD oder CVD, Streifen 23, 24 mit einem röntgensichtbaren Material in Form einer Beschichtung aufgebracht. Eine derart beschichtete Leiterplatte 22 ist aus Fig. 2 b) ersichtlich. Die Dicke der Beschichtung beträgt z. B. 50 µm und entspricht der späteren Dicke der Marker 15, 16. Die Streifen 23, 24 verlaufen senkrecht zur Längsrichtung. Die Streifen 23, 24 besitzen einen genau definierten Abstand 25 (z. B. Abstand der Mittellinien) z. B. von 15,5 mm und eine Breite 26 z. B. von 1 mm, welche sich durch das Beschichtungsverfahren exakt einstellen lassen. Der Abstand 25 und die Breite 26 der Streifen 23, 24 entsprechen dem späteren Abstand und der Breite der Marker 15, 16.

Von einer derart gefertigten Leiterplatte können mehrere einzelne Polymerbänder 14 durch Schnitte in Längsrichtung abgeschnitten werden. Wie in Fig. 2c) gezeigt, enthält ein solches Polymerband 14 je einen Teilbereich des ersten Streifens 23 und des zweiten Streifens 24. Durch die geeignete Dimensionierung der Leiterplatte 22 muss zur Abtrennung eines Polymerbandes von der Leiterplatte nur entlang einer einzelnen Schnittlinie 27 geschnitten werden. Die Breite des Polymerbandes kann einfach durch Positionierung der Schnittlinie 27 und entsprechende Variation der Breite des abgeschnittenen Bandes eingestellt und an die verschiedenen Katheterarten angepasst werden. Damit ist es möglich, mit einem einzigen Schnitt das Polymerband 14 in einer Größe zu erhalten, die für die Herstellung eines Ballonkatheters 10 eingesetzt werden kann. Alternativ können andere Trennverfahren für das Abtrennen des Polymerbandes 14 von der Leiterplatte 22 eingesetzt werden.

Eine alternative Ausführungsform der Erfindung ist in Fig. 3 abgebildet, bei der anstelle des Polymerbandes als Polymerträger eine Polymerträger-Hülse 30, z. B. bestehend aus Polyimid, verwendet wird. An der Außenseite der Polymerträger-Hülse 30 sind zwei Marker 31, 32 in Form einer Beschichtung mit einem röntgensichtbaren Material angeordnet. Vorzugsweise wird die Hülse anschließend in Richtung seiner Längsachse (d. h. longitudinal) geschlitzt. Ein solcher als Polymerträger-Hülse ausgeführter Polymerträger kann zur Herstellung eines Ballonkatheters einfach über einen Innenschaft des Ballonkatheters geschoben werden und liegt in vorteilhafter Weise in einer stabilen Form auf dem Innenschaft. Die Befestigung an dem Innenschaft erfolgt analog zu der Befestigung des Polymerbands.

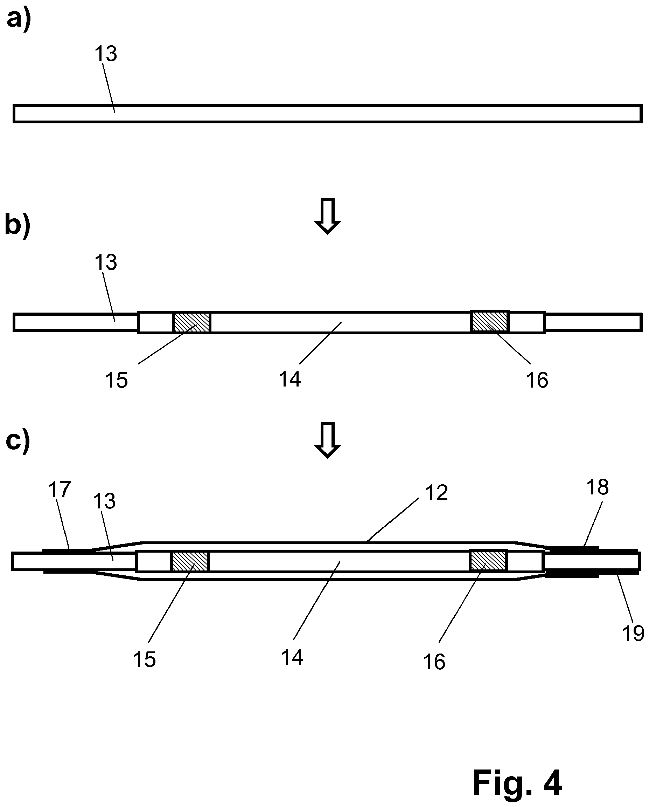

Fig. 4 a) bis 4 c) illustrieren die Herstellung eines erfindungsgemäßen Ballonkatheters 10 aus seinen Einzelteilen. Beginnend mit dem in Fig. 4 a) dargestellten Innenschaft 13 wird ein beispielsweise wie oben erläutert hergestelltes Polymerband 14 mit den Markern 15, 16 an dem Innenschaft 13 beispielsweise mittels Schweißen befestigt (siehe Fig. 4b).

In einem nächsten, in Fig. 4c) dargestellten Schritt wird der Ballon 12 um den Innenschaft 13 mit dem Polymerband 14 gelegt und mittels je einer Verschweißung an dem Innenschaft im distalen und proximalen Bereich 17, 18 des Innenschafts festgelegt.

Der mit diesem Verfahren hergestellte erfindungsgemäße Ballonkatheter 10 kann nun in ein Gefäß eingeführt werden. Durch die gegenüber herkömmlichen Markern verringerte Steifigkeit des Katheters im Bereich des Markers ist dies auch für schwer zugängliche Gefäße möglich. Da der erfindungsgemäße Katheter keine scharfen Kanten an den Markern aufweist, ist die Gefahr einer Perforation des Ballons durch einen Marker deutlich verringert. Weiter können die Marker durch oben beschriebene Herstellungsverfahren mit einer höheren Genauigkeit positioniert werden, so dass weniger Ausschuss bei der Produktion anfällt.

### Bezugszeichenliste

- 10: Ballonkatheter
- 12: Ballon
- 13: Innenschaft
- 14: Polymerträger-Band, kurz Polymerband
- 15: distaler Marker
- 16: proximaler Marker
- 17: distaler Bereich
- 18: proximaler Bereich
- 19: Außenschaft
- 22: Leiterplatte
- 23: erster Streifen
- 24: zweiter Streifen
- 25: Abstand der Marker
- 26: Breite der Marker
- 27: Schnittlinie
- 30: Polymerträger-Hülse
- 31: distaler Marker auf Polymerträger-Hülse
- 32: proximaler Marker auf Polymerträger-Hülse

## Patentansprüche

1. Ballonkatheter mit einem expandierbaren Ballon (12), einem in dem Ballon (12) angeordneten Innenschaft (13), und mindestens einem Marker (15, 16, 31, 32), wobei der mindestens eine Marker (15, 16, 31, 32) ein röntgensichtbares Material aufweist, **dadurch gekennzeichnet, dass** das röntgensichtbare Material als Beschichtung auf und/oder in einem Polymerträger (14, 30) ausgebildet ist und dass der Polymerträger (14, 30) auf dem Innenschaft (13) befestigt ist.

2. Ballonkatheter nach Anspruch 1, **dadurch gekennzeichnet, dass** ein erster Marker (15, 31) in einem ersten Abschnitt des Polymerträgers (14, 30) und ein zweiter Marker (16, 32) in einem zweiten Abschnitt des Polymerträgers (14, 30) angeordnet sind, wobei der erste Marker (15, 31) und der zweiter Marker (16,32) einen vorbestimmten Abstand (26) aufweisen.

3. Ballonkatheter nach Anspruch 1 oder 2, **dadurch gekennzeichnet, dass** der Polymerträger (14, 30) mit seinem proximalen Ende mittels einer Schweißverbindung am Innenschaft (13) befestigt und/oder der Polymerträger (14,30) mit seinem distalen Ende mittels einer Schweißverbindung am Innenschaft (13) befestigt ist.

4. Ballonkatheter nach Anspruch 1 oder 2, **dadurch gekennzeichnet, dass** der Polymerträger mittels einer Klebeverbindung auf dem Innenschaft befestigt ist.

5. Ballonkatheter nach einem der vorhergehenden Ansprüche, **dadurch gekennzeichnet, dass** der Polymerträger (14) platten- oder bandförmig ausgebildet ist und dass der Polymerträger (14) zumindest teilweise um den Innenschaft (13) gebogen ist.

6. Ballonkatheter nach einem der Ansprüche 1 bis 4, **dadurch gekennzeichnet, dass** der Polymerträger (30) hülsenförmig ausgebildet ist.

7. Ballonkatheter nach einem der vorhergehenden Ansprüche, **dadurch gekennzeichnet, dass** der Polymerträger (14, 30) Polyimid und/oder LCP enthält und/oder eine Dicke zwischen 10 und 50 µm, vorzugsweise zwischen 20 und 30 µm, aufweist.

8. Ballonkatheter nach einem der vorhergehenden Ansprüche, **dadurch gekennzeichnet, dass** das röntgensichtbare Material mindestens ein Material der Gruppe aufweisend Gold, Tantal, Niob, Platin und Legierungen aus diesen Materialien beinhaltet und/oder die Beschichtung eine Dicke zwischen 20 und 100 µm, vorzugsweise 40 und 60 µm, aufweist.

9. Verfahren zur Herstellung eines Ballonkatheters (10) nach Anspruch 1 mit den folgenden Schritten:
- Bereitstellen einer Polymerträger-Platte, eines Polymerträger-Bands (14) oder einer Polymerträger-Hülse (30),
- Auf- und/oder Einbringen einer Beschichtung aus einem röntgensichtbaren Material in Form mindestens eines Markers (15, 16, 31, 32) auf und/oder in der Polymerträger-Platte, dem Polymerträger-Band (14) oder der Polymerträger-Hülse (30) und
- Befestigen der beschichteten Polymerträger-Platte, des beschichteten Polymerträger-Band (14) oder der beschichteten Polymerträger-Hülse (30) auf dem Innenschaft des Ballonkatheters.

10. Verfahren nach Anspruch 9, **dadurch gekennzeichnet, dass** zum Befestigen eines vorzugsweise von einer Polymerträger-Platte (22) abgetrennten Polymerträger-Bands (14) auf dem Innenschaft (13) das Polymerträger-Band (14) zunächst auf dem Innenschaft (13) fixiert wird, dann der Innenschaft (13) innerhalb des Ballons (12) angeordnet wird und anschließend Ballon (12), Innenschaft (13) und Polymerband (14) an ihrem distalen Ende gleichzeitig miteinander verschweißt werden.

11. Verfahren nach Anspruch 8 oder 10, **dadurch gekennzeichnet, dass** die Polymerträger-Platte (22) eine Längsrichtung und eine dazu senkrechte Querrichtung aufweist, dass die Beschichtung mit dem röntgensichtbaren Material in der Form von mindestens zwei, in Querrichtung verlaufenden Streifen (23, 24) mit einem definiertem Abstand (25) erfolgt und dass das Polymerträger-Band (14) in Längsrichtung von der Polymerträger-Platte (22) abgetrennt wird.

12. Verfahren nach einem der Anspruch 9 bis 11, **dadurch gekennzeichnet, dass** das röntgensichtbare Material mittels einer Abscheidung aus der Flüssigphase oder der Gasphase, beispielsweise mittels PVD oder CVD, auf den Polymerträger (14, 22, 30) aufgebracht wird.
